(19) Europäisches Patentamt
European Patent Office
Office européen des brevets

(11) **EP 4 137 205 B1**

(12) **EUROPEAN PATENT SPECIFICATION**

(45) Date of publication and mention
of the grant of the patent:
**11.06.2025 Bulletin 2025/24**

(21) Application number: **21192314.9**

(22) Date of filing: **20.08.2021**

(51) International Patent Classification (IPC):
***A61N 5/10*** (2006.01)

(52) Cooperative Patent Classification (CPC):
**A61N 5/1031;** A61N 2005/1034; A61N 2005/1055

(54) **METHODS AND DEVICES FOR SIMULATING CHARGED-PARTICLE TRANSPORT IN AN EXTERNAL MAGNETIC FIELD, WITH APPLICATIONS TO RADIOTHERAPY PLANNING**

VERFAHREN UND VORRICHTUNGEN ZUR SIMULATION EINES LADUNGSTRÄGERTRANSPORTS IN EINEM ÄUSSEREN MAGNETFELD, MIT ANWENDUNGEN FÜR STRAHLENTHERAPIEPLANUNG

PROCÉDÉS ET DISPOSITIFS DE SIMULATION DE TRANSPORT DE PARTICULES CHARGÉES DANS UN CHAMP MAGNÉTIQUE EXTERNE, AVEC APPLICATIONS POUR LA PLANIFICATION DE RADIOTHÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.02.2023 Bulletin 2023/08**

(73) Proprietor: **RaySearch Laboratories AB**
**104 30 Stockholm (SE)**

(72) Inventors:
• **FÄLTH, Claes**
**752 35 Uppsala (SE)**
• **ANGERUD, Agnes**
**175 69 Järfälla (SE)**

(56) References cited:
CN-A- 107 050 667    US-A1- 2019 070 438
US-A1- 2019 175 940

• MALKOV V N ET AL: "Charged particle transport in magnetic fields in EGSnrc", MEDICAL PHYSICS, AIP, MELVILLE, NY, US, vol. 43, no. 7, 29 June 2016 (2016-06-29), pages 4447 - 4458, XP012209067, ISSN: 0094-2405, [retrieved on 20160629], DOI: 10.1118/1.4954318
• FIGUEROA R ET AL: "Theory, simulation and experiments for precise deflection control of radiotherapy electron beams", APPLIED RADIATION AND ISOTOPES, ELSEVIER, OXFORD, GB, vol. 141, 8 March 2018 (2018-03-08), pages 187 - 192, XP085502615, ISSN: 0969-8043, DOI: 10.1016/J.APRADISO.2018.03.004

**Description**

**TECHNICAL FIELD**

The present disclosure relates to the field of numerical simulation of charged-particle transport. It addresses a problem in the field of radiation therapy, namely, the generating of a radiation treatment plan to be delivered in the presence of an external magnetic field.

**BACKGROUND**

A treatment planning system is expected to be able to find a treatment plan that describes how to deliver a prescribed radiation dose to a tumor while minimizing radiation to surrounding tissue and organs at risk. For this purpose, the treatment planning system may compute what dose the patient will receive when a linear accelerator operates in accordance with a tentative treatment plan. Radiation therapy in the presence of a magnetic field is practiced, inter alia, in so-called magnetic resonance guided radiotherapy (MRgRT), wherein the magnetic field enables magnetic-resonance imaging (MRI) of the patient in real time. The external magnetic field is known to also influence the dose delivery to the patient's tissue (dose profile shift, electron return effect, electron streaming effect). It is furthermore being envisioned to utilize magnetic force for local dose adjustments; for example, a suitably localized magnetic field could deflect charged particles around a difficultly located organ-at-risk. It is therefore desirable to provide dose computation methods that account for magnetic effects as well.

Monte-Carlo dose computations are known to provide accurate approximations, and they are capable of a high level of detail in the material and a precise description of particle interactions. This class of dose computation methods are made possible by Monte-Carlo-based particle-transport simulations. Implementations of such dose computation methods may include particle-transport simulations as a component. An early method for simulating electron transport in the presence of electric and magnetic fields is presented in A. F. Bielajew, "Electron Transport in E and B Fields", in: T. M. Jenkins et al. (eds.), Monte Carlo Transport of Electrons and Photons, Ettore Majorana International Science Series, no. 38, Plenum Press, 1988. Here, the Lorentz force is integrated by evaluating the change in direction based on the direction and energy of the particle before the step (1-P approach). Bielajew's method is used in the softwares EGSnrc and GPUMCD.

A more recent Monte-Carlo-based particle transport simulation method which accounts for magnetic effects is disclosed in V. N. Malkov et al., "Charged particle transport in magnetic fields in EGSnrc", Med. Phys., vol. 43, no. 7, pp. 4447-4458 (2016). This is a three-point integration method (3-P approach) which incorporates multiple scattering when evaluating the change in direction.

Further, the PENELOPE code system uses an analytic solution with circular orbits. See F. Salvat et al., PENELOPE-2018: A Code System for Monte Carlo Simulations of Electron and Photon Transport, OECD Nuclear Energy Agency, NEA/MBDAV/R(2019)1 (2019).

One figure-of-merit by which the performance of particle-transport simulation methods may be compared is the maximum step lengths that they support under given conditions. Reference is made to equations 19.32-19.37 in the above-identified work by Bielajew, which introduce various dimensionless parameters which must stay small if convergence is to be expected. Generally, the desired smallness of the parameters can be achieved by reducing the step length, but this will bring about a corresponding computational load increase for simulating a particle path of a given length. With respect to a parameter $\delta$ representing the direction change (in radians) per step, it has been shown that 3-P integration could perform satisfactorily up to $\delta = 0.2$, while 1-P integration is typically limited to the much smaller $\delta \leq 0.02$. The limitation $\delta \leq 0.02$ is recommended by both the Malkov (2016) paper and PENELOPE.

**SUMMARY**

It is an objective of the present disclosure to make methods and devices available for accurately and efficiently simulating charged-particle transport in an external magnetic field. In particular, the methods and devices should be able to run with an acceptably large step length. Another objective is to propose such methods and devices that are suitable for supporting radiotherapeutic dose calculations. A further objective is to address simulation of charged-particle transport governed by a nonlinear equation of motion. It is a still further objective of this disclosure to propose a method for computing a dose distribution in a medium to be irradiated according to a treatment plan comprising machine-oriented instructions.

At least some of these objectives are achieved by the invention as defined by the independent claims. The dependent claims relate to advantageous embodiments of the invention.

In a first aspect of the present invention, there is provided a method of simulating charged-particle transport in an external magnetic field **B** according to a model where Newton's second law for the unit direction vector of a charged particle,

$$\boldsymbol{u}'(t) = \boldsymbol{F}\big(\boldsymbol{u}(t), \eta(t)\big),$$

comprises a force term $\boldsymbol{F}$ which depends on the unit direction vector $\boldsymbol{u}(t)$ and further depends nonlinearly on a motion variable $\eta(t)$ of the particle. In this method, Newton's second law is solved using an implicit finite-difference method in which, for the duration $\Delta t$ of a time step, the force term is approximated by its value for interpolated values of the unit direction vector and the motion variable. The interpolated value of each quantity is a combination of that quantity's values at the endpoints of the time step, $t$ and $t + \Delta t$.

An important benefit of this method is that it can be operated over a large range of step sizes. More precisely, numerical evaluation suggests that it does not suffer any noticeable loss of accuracy even when the direction change per time step is as large as $\delta = 0.3$ radians. Further, the proposed method according to the first aspect can readily be combined with others, such as the random hinge method used in the applicant's RayStation™ software and in the PENELOPE code system. It could also be applied to each individual point in the 3-P approach, possibly to enable larger step sizes.

In some embodiments, the interpolated values to be used in approximating the force term are linear combinations of the endpoint values. In particular, the interpolated values may be mean values, whereby:

$$\frac{\Delta \boldsymbol{u}}{\Delta t} = \boldsymbol{F}\left(\frac{\boldsymbol{u}(t + \Delta t) + \boldsymbol{u}(t)}{2}, \frac{\eta(t + \Delta t) + \eta(t)}{2}\right).$$

Each of these options provides a simple and robust approximation of Newton's second law.

In some embodiments, the simulation method further comprises simulating particle interaction, such as multiple scattering between the particle and the medium. The interaction is simulated as a discrete event occurring between two time steps of the finite-difference method.

The charged particle for which transport is simulated can be any charged particle or nucleus, possibly including positrons, pions and muons. In some embodiments, the charged particle is an electron, a proton, a helium ion or a carbon ion.

The method according to the first aspect may be integrated into a dose distribution computation method, which takes as its input a treatment plan by which a medium is to be irradiated by charged particles in the presence of a magnetic field. The method includes simulating particle transport including interaction with the medium, wherein the dose distribution is computed as a result of the simulated interactions.

In a second aspect of the present invention, there is provided a treatment planning system configured to execute the above method. The invention further relates to a computer program containing instructions for causing a computer, or the treatment planning system in particular, to carry out the above method. The computer program may be stored or distributed on a data carrier. As used herein, a "data carrier" may be a transitory data carrier, such as modulated electromagnetic or optical waves, or a non-transitory data carrier. Non-transitory data carriers include volatile and non-volatile memories, such as permanent and non-permanent storage media of magnetic, optical or solid-state type. Still within the scope of "data carrier", such memories may be fixedly mounted or portable.

The present disclosure refers to "Newton's second law" and its "force term" in a generalized sense. In some embodiments, the differential equation solved is not the classical form of Newton's second law, in terms of velocity v(t) in units of 1 m/s, but rather Newton's second law formulated for the unit direction vector

$$\boldsymbol{u}(t) = \frac{\boldsymbol{v}(t)}{|\boldsymbol{v}(t)|},$$

which quantity may have the unit 1/s. The classical form of Newton's second law may be transformed into the unit-direction-vector formulation by a variable substitution; see Bielajew (1988). In this formulation, while the right-hand side of Newton's second law has different units than classical force, it is nevertheless referred to as a force term.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order described, unless explicitly stated.

## BRIEF DESCRIPTION OF THE DRAWINGS

Aspects and embodiments are now described, by way of example, with reference to the accompanying drawings, on which:

figure 1 is a flowchart of a method of computing a dose distribution according to an embodiment;

figure 2 is a block diagram of a treatment planning system;

figure 3 shows a detail of a radiation delivery system;

figure 4 shows a multi-leaf collimator suitable for shaping a radiation beam;

figures 5 and 6 are two projections of an electron trajectory simulated according to a simplified model and using a one-point (1-P) and a two-point (2-P) integration method of equal step size $\delta = 0.2$, which are compared to a small-step 1-P integration understood to be ground truth;

figure 7 is a differential dose distribution showing the extent to which a dose computed by 1-P integration with $\delta = 0.3$ differs from a ground-truth dose distribution (computed by small-step 1-P integration) shown in figure 9;

figure 8 is a further differential dose distribution showing the extent to which a dose computed by 2-P integration with $\delta = 0.3$ according to an embodiment of the present invention differs from the same ground-truth dose distribution; and

figure 9 shows the ground-truth dose distribution to which figures 7 and 8 refer.

## DETAILED DESCRIPTION

The aspects of the present disclosure will now be described more fully hereinafter with reference to the accompanying drawings, on which certain embodiments of the invention are shown. These aspects may, however, be embodied in many different forms and should not be construed as limiting; rather, these embodiments are provided by way of example so that this disclosure will be thorough and complete, and to fully convey the scope of all aspects of invention to those skilled in the art. Like numbers refer to like elements throughout the description.

One of the intended applications of the charged-particle transport simulation method according to present invention is in a dose calculation method 100, which may be adapted for MRgRT or magnetically enhanced dose delivery. Another application of the charged-particle transport simulation method is to calculate trajectories of electrons travelling in magnetic fields which have been generated for macroscopic deflection purposes, e.g., around organs-at-risk. For this second purpose, charged particles entering the human body with an energy in the range 150-200 MeV may be deflected by a magnetic flux density of about 5-10 T.

The method 100 can be used to calculate a dose distribution in a medium to be irradiated with charged or neutral particles according to a treatment plan. For example, the medium may be exposed to external photon radiation, and charged particles are released as the photons interact with the medium. When the irradiation takes place in the presence of an external magnetic field **B**, a Lorenz force acts on each charged particle. The charged particles for which transport is simulated can be any charged particles or nuclei. For instance, the charged particles may be electrons, protons, helium ions or carbon ions.

The calculated dose distribution may be expressed in terms of dose as a function of location, wherein the medium is partitioned into a dose grid, such as a three-dimensional grid of voxels with a size of 1 to 5 mm, preferably less than 2 mm.

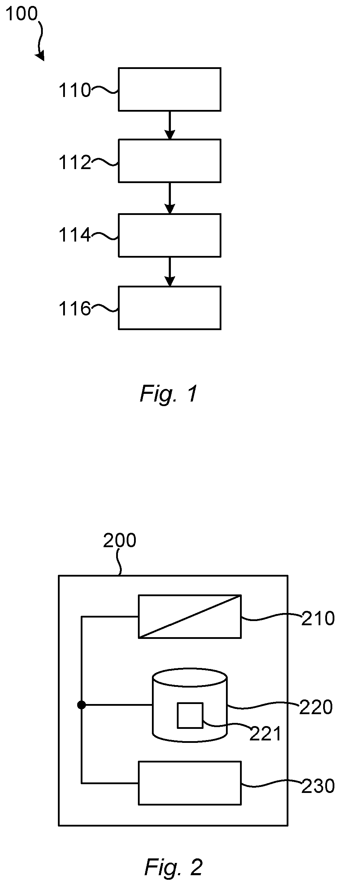

The method 100, which is visualized as a flowchart in figure 1, may be executed on a general-purpose computer, a combination of computers or a dedicated processing system. In particular, the method 100 may be implemented in a radiotherapeutic treatment planning system.

An example treatment planning system 200 is depicted, in block-diagram form, in figure 2. The treatment planning system 200 comprises an interface 210, a memory 220 and processing circuitry 230. Data communication between these components is possible over an internal data bus or, in distributed implementations, a data network. In such distributed implementations, the components of the treatment planning system 200 may be provided as networked ("cloud") resources. The interface 210 is configured for communication with external parties, such as other processors or a human operator. Using the interface 210, the treatment planning system 200 may obtain a treatment plan for which the dose distribution is to be computed, output the computed dose distribution for review by the operator, receive configuration input from the operator, and so forth. Needless to say, if the treatment plan has been generated in the treatment planning system 200, it can be transferred internally and need not pass by the interface 210. The memory 220 may store configuration settings, data descriptive of the radiation delivery system 300, data descriptive of the patients and their prescribed doses, as well as program code 221 executable by the processing circuitry 230. The processing circuitry 230 can comprise one or more processors. For instance, it include a central processing unit (CPU), a graphics processing unit (GPU) and/or a hardware accelerator.

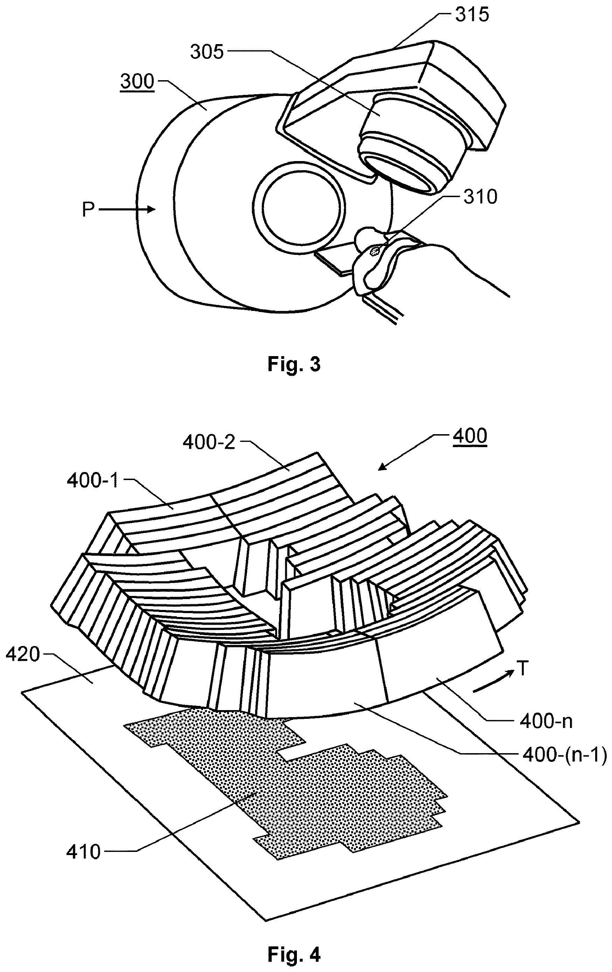

Within the method 100, a first step 110 includes obtaining a treatment plan comprising instructions suitable for controlling a radiation delivery system 300 (see figure 3). The instructions in the treatment plan may be machine-oriented (or machine-level) instructions, which define the behavior of actuators in the radiation delivery system 300, including actuators associated with a radiation source, a radiation source gantry, a rotatable patient couch or the like. The instructions in the treatment plan can normally be executed by these actuators or by control circuitry in the radiation delivery system 300 without substantive changes. Typically, the instructions in the treatment plan are not expressed in terms of clinical effects, and not in terms of the delivered dose. To compute the dose which is delivered to the medium when the instructions are executed, the method 100 may be relied upon. A possible use of the method 100 is to compute the dose distribution which would result if a tentative treatment plan was carried out, e.g., as a validation of the treatment plan. If the computed dose distribution is not satisfactory, the treatment plan may need to be modified or generated anew with adjusted settings.

In step 114, a particle transport simulation is performed. The particle transport simulation may be a computer-implemented Monte-Carlo-type simulation. On a general level, the simulation includes sampling an incident particle (i.e., a neutral or charged particle impinging on the medium to be irradiated), simulating interactions between the medium and the particle, and simulating further interactions between the medium and further particles created by said interactions. The incident particle, including its position direction and/or energy, is sampled in accordance with the treatment plan. In some implementations, the incident particle is not sampled directly from the treatment plan but from a fluence field, which has been calculated on the basis of the treatment plan in a foregoing optional step 112. (The concept of a fluence field is exemplified in the description of figure 4.) In some implementations, the particle transport simulation 114 may include the following substeps:

- Transporting the particle, which was sampled in accordance with the treatment plan, to the boundary where it enters the dose grid.

- Sampling the distance of the next interaction and transporting the particle there.

- Sampling which type of interaction is to take place, based on the total cross sections. The interaction may be multiple scattering. The total effect of multiple scattering over a distance travelled by the particle may be simulated as a discrete interaction event (condensed history).

- Initiating, in the simulation model, any new particles that are created in the interaction (catastrophic event) and sampling the energy and direction of the particles after the interaction based on the differential cross sections. The initiating may include instantiating data structures representing the new particles.

- Putting the newly initiated particles on a stack for later simulation.

- Continuing transporting the particle until it has lost all energy or leaves the dose grid.

In a next step 116, a dose distribution resulting from the simulated interactions is computed. More precisely, the energy loss of an electron as it moves through a voxel may be deposited in the voxel and converted to a dose based on the density of the material in the voxel. In some embodiments, special care is taken at voxel boundaries. For example, the bookkeeping relating to energy deposition may be simpler (or inconsistencies avoided) if the step length is adjusted to avoid occasional voxel-border crossings. Further details are found in the article by Malkov (2016).

The techniques for simulating charged-particle transport according to the present invention are put to use in the step 114, and more precisely when the particles to be transported are charged. The techniques can be characterized as two-point (2-P) integration methods which take into account the energy loss and the magnetic field bending (or deflection) when calculating the magnetic field deflection in one simulation step. Similar to some existing approaches, multiple scattering and other interactions are treated separately from the magnetic field bending. Since however the two-point integration is more accurate than one-step methods, the magnetic field bending size can be made larger without any noticeable loss of accuracy. It follows from results in the above-cited work by Bielajew that the method to be described is valid if the step is short enough such that (i) the energy loss is small, (ii) the magnetic field is approximately constant, and (iii) the change in direction is minimal. If small energy loss and constant magnetic field are assumed, the admissible step length for an angular change of $\delta$ radians per step is

$$s(\delta) = \frac{\beta(t)E(t)}{|q|cB} \frac{1}{|\boldsymbol{u}(t) \times \widehat{\boldsymbol{B}}|} \delta$$

where q is the particle's charge, c is the speed of light and the magnetic field has been decomposed as $\boldsymbol{B} = B\hat{\boldsymbol{B}}$ with

$$\left|\hat{\boldsymbol{B}}\right| = |(b_1, b_2, b_3)| = 1.$$

Numerical evaluations for a phantom have been performed for step lengths corresponding to $\delta = 0.01, 0.02, 0.05, 0.1, 0.15, 0.2$ and $0.3$, and satisfactory accuracy was obtained up to at least $\delta = 0.2$. For a step length of s, the simulation step will have a duration given by

$$\Delta t = \frac{s}{2c\beta(t)} \left(1 + \frac{\beta(t)}{\beta(t + \Delta t)}\right),$$

where $\beta(t)$ is the particle velocity at time $t$ in units of the speed of light c.

According to an embodiment, Newton's second law for the unit direction vector $\boldsymbol{u}(t)$ of the charged particle,

$$\boldsymbol{u}'(t) = \boldsymbol{F}\big(\boldsymbol{u}(t), \eta(t)\big),$$

is solved by an implicit finite-difference method, in which the force term $\boldsymbol{F}(\boldsymbol{u}(t), \eta(t))$ is approximated by a constant value for the duration of a time step $\Delta t$. The force term depends on the unit direction vector $\boldsymbol{u}(t)$ and a motion variable $\eta(t)$. More precisely, the force term is approximated by its value for interpolated values of the endpoint values ($t$ and $t + \Delta t$ are the endpoints of a time step) of the unit direction vector and the motion variable, as follows:

$$\frac{\Delta \boldsymbol{u}}{\Delta t} = \boldsymbol{F}\left(p\big(\boldsymbol{u}(t + \Delta t), \boldsymbol{u}(t)\big), p\big(\eta(t + \Delta t), \eta(t)\big)\right),$$

where $\Delta\boldsymbol{u}$ represents the magnetic bending (deflection) such that $\boldsymbol{u}(t + \Delta t) = \boldsymbol{u}(t) + \Delta\boldsymbol{u}$. In this expression, $p(x, y)$ denotes an interpolation function by which the arguments $x$ and $y$ are combined linearly or nonlinearly. In one embodiment, the interpolation function is a linear combination of the arguments:

$$p(x, y) = \frac{p_1 x + p_2 y}{p_1 + p_2},$$

where $p_1, p_2$ are arbitrary positive constants. Here, $p_1 \neq p_2$ or $p_1 = p_2$. In the latter case, the interpolation function maps the arguments $x, y$ to their arithmetic mean:

$$\frac{\Delta \boldsymbol{u}}{\Delta t} = \boldsymbol{F}\left(\frac{\boldsymbol{u}(t + \Delta t) + \boldsymbol{u}(t)}{2}, \frac{\eta(t + \Delta t) + \eta(t)}{2}\right).$$

For nonlinear $\boldsymbol{F}$, this approximation differs markedly from the one used in the classical Heun method, namely:

$$\frac{\Delta \boldsymbol{u}}{\Delta t} = \frac{\boldsymbol{F}\big(\boldsymbol{u}(t + \Delta t), \eta(t + \Delta t)\big) + \boldsymbol{F}\big(\boldsymbol{u}(t), \eta(t)\big)}{2}.$$

The motion variable $\eta(t)$ may be total energy, kinetic energy, linear momentum or velocity. If the force term is equal to

$$\frac{qBc^2}{E(t)} \boldsymbol{u}(t) \times \hat{\boldsymbol{B}},$$

then the total energy $E(t)$ can be identified as the motion variable $\eta(t)$. According to the implicit finite-difference method of the present embodiment, Newton's second law is approximated as

$$\frac{\Delta \boldsymbol{u}}{\Delta t} = \boldsymbol{F}\left(\frac{\boldsymbol{u}(t+\Delta t)+\boldsymbol{u}(t)}{2}, \frac{E(t+\Delta t)+E(t)}{2}\right) = qBc^2\left(\frac{\boldsymbol{u}(t+\Delta t)+\boldsymbol{u}(t)}{E(t+\Delta t)+E(t)}\right) \times \widehat{\boldsymbol{B}}.$$

This expression will be simplified by introducing the notation

$$\boldsymbol{u}(t) = \boldsymbol{u}_\| + \boldsymbol{u}_\perp,$$

$$\boldsymbol{u}(t+\Delta t) = \boldsymbol{u}_\| + (\boldsymbol{u}_\perp + \Delta \boldsymbol{u})N',$$

$$N' = \frac{|\boldsymbol{u}_\perp|}{|\boldsymbol{u}_\perp + \Delta \boldsymbol{u}|},$$ where the component $\boldsymbol{u}_\|$ is parallel to the magnetic field $\widehat{\boldsymbol{B}}$, which yields:

$$\Delta \boldsymbol{u} = qc^2 B\Delta t \frac{\boldsymbol{u}_\| + N'(\boldsymbol{u}_\perp + \Delta \boldsymbol{u}) + \boldsymbol{u}_\| + \boldsymbol{u}_\perp}{E(t+\Delta t)+E(t)} \times \widehat{\boldsymbol{B}}.$$

The cross product is rewritten in matrix form as $\boldsymbol{u} \times \widehat{\boldsymbol{B}} = \tilde{B}\boldsymbol{u}$ with

$$\tilde{B} = \begin{bmatrix} 0 & b_3 & -b_2 \\ -b_3 & 0 & b_1 \\ b_2 & -b_1 & 0 \end{bmatrix}.$$

(The symbol $\boldsymbol{u}$ is used for the unit direction vector components as well as their column-matrix representations.) As a result of several cancellations and of grouping the coefficients as

$$\alpha = \frac{qBc^2\Delta t}{E(t+\Delta t)+E(t)},$$

one obtains:

$$\Delta \boldsymbol{u} = \alpha(1+N')\tilde{B}\boldsymbol{u}_\perp + \alpha N\tilde{B}\Delta \boldsymbol{u} = \left(I - \alpha N'\tilde{B}\right)^{-1}\alpha(1+N')\tilde{B}\boldsymbol{u}_\perp,$$

where $I$ is the identity matrix. Inserting finally $N' = 1$ (particle energy conserved under Lorenz force) and reidentifying the cross product, this simplifies as

$$\Delta \boldsymbol{u} = \frac{2\alpha}{1+\alpha^2}\left(\boldsymbol{u}_\perp \times \widehat{\boldsymbol{B}} - \alpha\boldsymbol{u}_\perp\right)$$

or, equivalently,

$$\boldsymbol{u}(t+\Delta t) = \boldsymbol{u}(t) + \frac{2\alpha}{1+\alpha^2}\left(\boldsymbol{u}_\perp \times \widehat{\boldsymbol{B}} - \alpha\boldsymbol{u}_\perp\right).$$

It has been shown in the above-cited works that the new position of the electron can be computed from the deflection $\Delta \boldsymbol{u}$ as

$$\boldsymbol{x}(t+\Delta t) = \boldsymbol{x}(t) + s\left(\boldsymbol{u}(t) + \frac{1}{4}\left(1 + \frac{\beta(t)}{\beta(t+\Delta t)}\right)\Delta \boldsymbol{u}\right).$$

At this stage, it is optional to apply the following normalization:

$$\boldsymbol{u}(t + \Delta t) \leftarrow \boldsymbol{u}_{\parallel} + \frac{\boldsymbol{u}_{\perp} + \Delta \boldsymbol{u}}{|\boldsymbol{u}_{\perp} + \Delta \boldsymbol{u}|} |\boldsymbol{u}_{\perp}|$$

This limits the propagation of floating-point imprecision. It is recalled that the parallel and perpendicular components refer to $\boldsymbol{u}(t)$.

The value $E(t + \Delta t)$ of the total energy at the later endpoint, which appears in $\alpha$, can for example be computed by evaluating a stopping-power function for the distance s travelled by the particle during the time step. In the present disclosure, the stopping power $S(T)$ at kinetic energy $T$ is defined as the mean energy loss per unit path length, and it can be modeled as

$$S(T) = N \int W \left( \frac{d\sigma(T)}{dW} \right) dW,$$

where $N$ is the number of scattering targets per unit volume and $\frac{d\sigma(T)}{dW}$ is the differential cross section for an interaction with energy loss $W$. The stopping power can be divided into collision stopping power and radiative stopping power, wherein the collision stopping power dominates the other contribution for particle energies up to 10 MeV. Reference is made to M. J. Berger, "Electron stopping powers for transport calculations", in: T. M. Jenkins et al. (eds.), Monte Carlo Transport of Electrons and Photons, Ettore Majorana International Science Series, no. 38, Plenum Press, 1988. In embodiments of the present invention, the energy loss may be modeled as restricted collision stopping power.

Figure 3 schematically illustrates an example of a radiation delivery system (or radiation therapy machine) 300, which is configured to operate in accordance with a radiation treatment plan P, which may have been determined by a treatment planning system. As mentioned initially, the treatment plan P may have been generated with an aim of causing the prescribed radiation dose to be delivered to the at least one target while delivering not more than a threshold amount of radiation to any nearby organs-at-risk. The radiation delivery system 300 is configured to carry out therapy in respect of a treatment volume 310 in a patient's body by controlling at least one radiation source 305 of the system 300 in accordance with the treatment plan P. The radiation source 305 may for instance be a linear accelerator. It may be integrated in a gantry 315.

The radiation delivery system 300 may further include means for generating a magnetic field with a definite orientation. To mention one example, the magnetic field can be parallel to a ray direction of the radiation source 305. In another example, the magnetic field is oriented along the patient's longitudinal axis (foot to head).

Figure 4 shows an example multi-leaf collimator (MLC) 400, which may be used in the radiation delivery system 300 as a blocking device to control the radiation from the at least one radiation source 305 therein. The MLC 400 has a set of collimator leaves 400-1, 400-2, ..., 400-(n-1), 400-n, which are arranged as opposing pairs. Typically, each leaf has a width of between 5 mm and 10 mm. The MLC 400 is arranged in the path of a radiation field, and by selectively shifting the collimator leaves 400-1, 400-2, ..., 400-(n-1), 400-n in the direction of travel T, a shape of an exposed fluence region 410 for the field on a beam plane 420 can be altered. The leaf adjustments can be effectuated continuously or in discrete steps. The fluence transmitted through an opening in the MLC 400 is uniform at a given instance in time. However, a modulated fluence profile in the beam plane 420 can be generated by superposition in time of multiple fluences with different shapes of the opening in the MLC 400.

In dynamic multi-leaf collimation (DMLC) therapy, which the invention is applicable to, the collimator leaves 400-1, 400-2, ... , 400-(n-1), 400-n are moved during irradiation. In segmental multi-leaf collimation (or so-called step-and-shoot) treatment, which the invention is also applicable to, the collimator leaves 400-1, 400-2, ..., 400-(n-1), 400-n are stationary during irradiation. Here, the radiation beam is switched off when the collimator leaves 400-1, 400-2, ..., 400-(n-1), 400-n are being repositioned.

Intensity-modulated radiation therapy (IMRT) and volumetric-modulated arc therapy (VMAT) treatments are two important applications of DMLC treatment. An IMRT treatment is composed of a set of static beams that are delivered consecutively. The irradiation is here switched off as the gantry 315 holding the at least one radiation source 305 is rotated into position for a next beam. Treatment delivery for VMAT is performed with the gantry 315 continuously rotating during irradiation. Consequently, the therapeutic fluence is here instead delivered over an arc of up to 360 degrees. In VMAT, a radiation treatment plan can be composed of several, possibly overlapping, arcs. Both IMRT and VMAT treatments are normally delivered over a set of identical treatment fractions, e.g., thirty daily fractions in the course of six weeks.

Ion-based radiation treatments involve irradiating a patient with protons or heavier ions. This is often done in the form of Pencil Beam Scanning (PBS), which involves delivering radiation to the patient in the form of beams from different beam directions. Each beam includes a number of spots, up to several thousands, where each spot is defined by its position, direction, weight and energy. The beam direction depends on the gantry position and angle, as well as the position and orientation of the couch. The direction of each spot may be adjusted by the use of magnets before entering the patient, so that the beam will cover a larger area of the patient. The spot weight indicates the number of particles included in the spot, and the energy determines how far the particles will travel in the treatment volume 310. The direction and energy therefore determine where the Bragg peak of this spot will be located within the patient, that is, where most of its energy will be deposited.

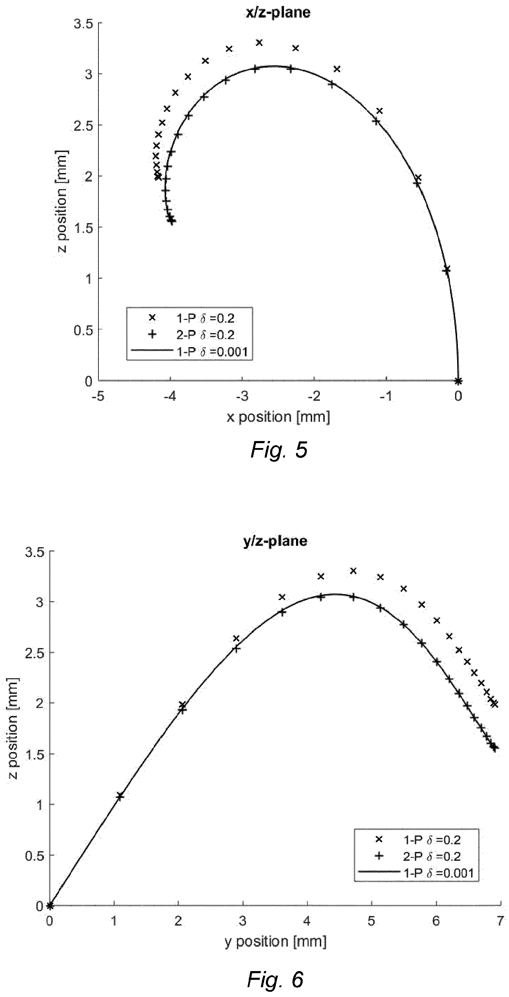

Figure 5 is a view in the *x-z* plane of an electron trajectory simulated according to a simplified model where only energy loss based on the stopping power for water and angular deflection due to the Lorenz force from a 1.5 T magnetic field in the *y* direction are accounted for. The electron was initiated with a kinetic energy of 2 MeV with

$$u(0) = \frac{1}{\sqrt{2}}(0,1,1)$$

. The electron was stepped using the 1-P integration method with $\delta$ = 0.2 and, according to an embodiment of the present invention, using the 2-P integration method, also with $\delta$ = 0.2. The results are plotted using symbols $\times$ and +, respectively. Plotted in solid line is a calculation using the 1-P method with $\delta$ = 0.001. Since the 1-P method is known to perform well in the 'easy' case of very small step length (i.e., when the particles are transported with very small increments), these results may be considered as a representative benchmark or ground truth. It is seen in figure 5 that the trajectory simulated with the 2-P method differs considerably less from the ground-truth trajectory. It may be hypothesized, therefore, that the 2-P method can be run at considerably larger step size $\delta$ than the conventional 1-P method without any perceptible loss of accuracy.

Figure 6 is a view in the *y-z* plane of the same simulated electron trajectories. This appears to confirm the favorable impression from figure 5 of the performance of the inventive 2-P method.

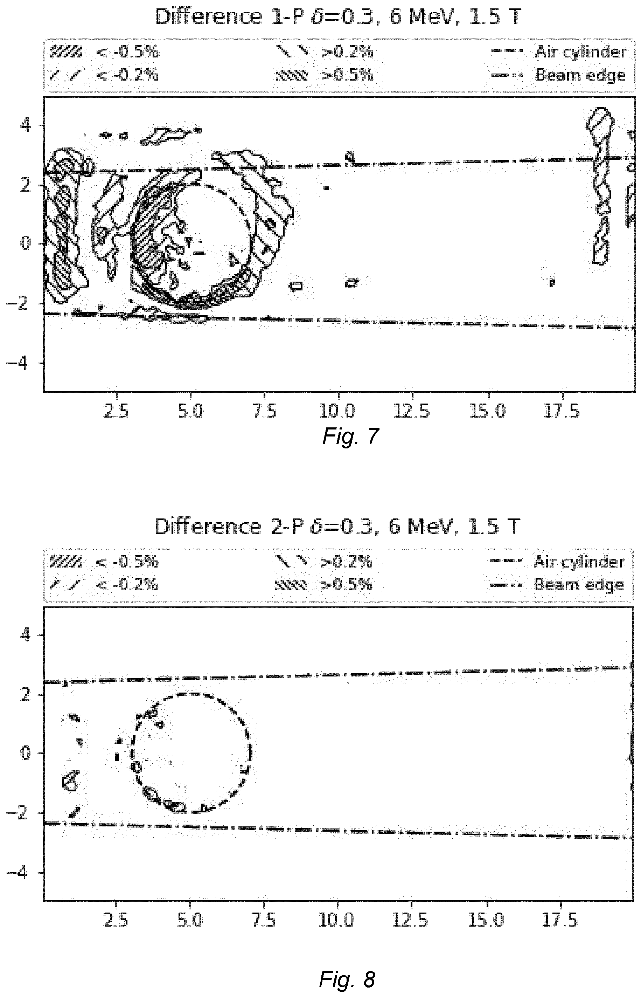

Figures 7 to 9 relate to simulations for a phantom consisting of a 20 $\times$ 20 $\times$ 20 cm cube of water with a cylinder of air inside. The air cylinder has a radius of 2 cm, is located 8 cm above (*x*) and 3 cm to the side of (*z*) the origin, and the longitudinal direction of the cylinder is in the magnetic field direction. The idea behind the phantom was to evaluate how the different simulations compare around the air cavity. Differences in the dose computations were expected to occur near the air cavity due to the sudden shift in medium density. The simulations were performed with the air cavity phantom using a 5 $\times$ 5 cm field with incident energies of 0.5, 2 and 6 MeV and a magnetic flux density both 1.5 and 0.35 T. The statistical uncertainty of the simulations was set to 0.1% to minimize the stochastic noise, such that only systematic differences were observed. The simulations were compared by visually inspecting the differences between the simulations with $\delta$ = 0.01 and the simulations with $\delta$ = 0.3.

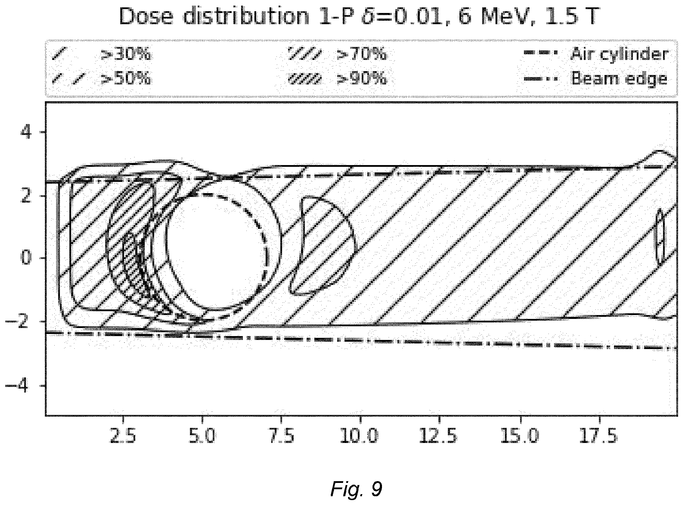

The results of the comparisons for 6 MeV and 1.5 T can be seen in figures 7 and 8. It appears that the 1-P integration with large $\delta$ (figure 7) introduces systematic differences compared to the ground-truth results (figure 9), whereas the 2-P integration with equal $\delta$ (figure 8) does not. The deficiencies of the 1-P method are most pronounced in high-gradient areas, at the build-up of the dose where the beam enters the notional patient, in and around the air cavity and at the edges of the beam. The differences are of the order of 1% or less in high-gradient areas, which might have been acceptable. However, the 2-P integration hardly produces any systematic differences, only some seemingly random differences in the air cavity, which may be attributed to stochastic noise, and some overestimation just inside the air region. One may conclude that that the 2-P integration method can be used with larger $\delta$ values than the 1-P integration method without affecting the accuracy of the simulation. This can drastically improve the computation time of the simulations compared to the 1-P integration method with a conservatively chosen $\delta$ = 0.02, which is the recommended value of the EGSnrc implementation in Malkov (2016).

The aspects of the present disclosure have mainly been described above with reference to a few embodiments. However, as is readily appreciated by a person skilled in the art, other embodiments than the ones disclosed above are equally possible within the scope of the invention, as defined by the appended patent claims.

**Claims**

1.  A computer-implemented method for supporting a radiotherapeutic dose calculation, the method configured for simulating charged-particle transport in an external magnetic field **B** according to a model where Newton's second law for the unit direction vector of a charged particle, $u'(t) = F(u(t), \eta(t))$, comprises a force term F which depends on the unit direction vector $u(t)$ and further depends non-linearly on a motion variable $\eta(t)$ of the particle, the method comprising: solving Newton's second law using an implicit finite-difference method in which, for the duration of a time step, the force term is approximated by its value for interpolated values of the unit direction vector and the motion variable, wherein the interpolated value of each quantity is a combination of that quantity's values at the end points $t, t + \Delta t$ of the time step.

2. The method of claim 1, wherein at least one of the interpolated values is a linear combination of the end point values.

3. The method of claim 2, wherein at least one of the interpolated values is a mean of the end point values.

4. The method of any of the preceding claims, wherein said at least one motion variable $\eta(t)$ of the particle is one or more of: total energy, kinetic energy, linear momentum, velocity.

5. The method of claim 4, wherein the value of the motion variable at the later end point, $\eta(t + \Delta t)$, is computed by evaluating a stopping-power function for the distance travelled by the particle.

6. The method of claim 4 or 5, wherein the motion variable is total energy $E(t)$, and the force term is equal to

$$F\big(\boldsymbol{u}(t), \eta(t)\big) = \frac{qBc^2}{E(t)}\, \boldsymbol{u}(t) \times \widehat{\boldsymbol{B}},$$

where $q$ is the particle's charge and $\hat{\boldsymbol{B}}$ is a unit vector such that $\boldsymbol{B} = B\hat{\boldsymbol{B}}$.

7. The method of claim 6, wherein the finite-difference method evolves the unit direction vector as follows:

$$\boldsymbol{u}(t + \Delta t) = \boldsymbol{u}(t) + \frac{2\alpha}{1 + \alpha^2}\big(\boldsymbol{u}_\perp \times \widehat{\boldsymbol{B}} - \alpha \boldsymbol{u}_\perp\big),$$

where $\boldsymbol{u}_\perp$ is the unit direction vector's component perpendicular to the magnetic field and

$$\alpha = \frac{qBc^2 \Delta t}{E(t + \Delta t) + E(t)}.$$

8. The method of any of the preceding claims, further comprising:
simulating a discrete interaction event occurring between two time steps of the finite-difference method.

9. The method of claim 8, wherein the simulated interaction includes multiple scattering.

10. The method of any of the preceding claims, wherein the charged particle is an electron, a proton, a helium ion or a carbon ion.

11. The method of any of the preceding claims, which is executed as part of particle transport simulation in a medium to be irradiated according to a radiotherapeutic treatment plan.

12. A method (100) of computing a dose distribution in a medium to be irradiated according to a treatment plan, the method comprising:

obtaining (110) a treatment plan comprising instructions suitable for controlling a radiation delivery system (300);
performing (114) a particle transport simulation, including:

- sampling an incident particle in accordance with the treatment plan,
- simulating interactions between the medium and the particle, and
- simulating further interactions between the medium and further particles created by said interactions,

computing (116) a dose distribution resulting from the simulated interactions,
wherein said particle transport simulation (114) includes executing the method of any of the preceding claims.

13. The method of claim 12, wherein the particle transport simulation is a Monte-Carlo-type simulation.

14. The method of claim 12 or 13, further comprising:

computing (112) a fluence field on the basis of the treatment plan,
wherein the incident particle is sampled based on the fluence field.

15. A treatment planning system (200) comprising memory (220) and processing circuitry (230) configured to perform the method of any of the preceding claims.

16. A computer program (221) comprising instructions which, when the program is executed by a computer, cause the computer to carry out the method of any of claims 1 to 14.

**Patentansprüche**

1. Computerimplementiertes Verfahren zum Unterstützen der Berechnung einer strahlentherapeutischen Dosis, wobei das Verfahren zum Simulieren eines Transports von geladenen Teilchen in einem externen Magnetfeld **B** konfiguriert ist, gemäß einem Modell, in dem das zweite Newtonsche Gesetz für den Einheitsrichtungsvektor eines geladenen Teilchens $u'(t) = F(u(t), \eta(t))$ einen Kraftterm $F$ umfasst, der von dem Einheitsrichtungsvektor $u(t)$ abhängt, und ferner nicht linear von einer Bewegungsvariablen $\eta(t)$ des Teilchens abhängt, das Verfahren umfassend:
Lösen des zweiten Newtonschen Gesetzes unter Verwendung eines impliziten Finite-Differenzen-Verfahrens, bei dem für die Dauer eines Zeitschritts der Kraftterm durch seinen Wert für interpolierte Werte des Einheitsrichtungsvektors und der Bewegungsvariablen angenähert wird, wobei der interpolierte Wert jeder Menge eine Kombination der Werte dieser Menge an den Endpunkten $t, t + \Delta t$ des Zeitschritts ist.

2. Verfahren nach Anspruch 1, wobei mindestens einer der interpolierten Werte eine lineare Kombination der Endpunktwerte ist.

3. Verfahren nach Anspruch 2, wobei mindestens einer der interpolierten Werte ein Mittel der Endpunktwerte ist.

4. Verfahren nach einem der vorstehenden Ansprüche, wobei die mindestens eine Bewegungsvariable $\eta(t)$ des Teilchens eines oder mehrere ist von: Gesamtenergie, kinetischer Energie, linearem Impuls, Geschwindigkeit.

5. Verfahren nach Anspruch 4, wobei der Wert der Bewegungsvariablen an dem späteren Endpunkt $\eta(t + \Delta t)$ berechnet wird, durch Auswerten eine Stoppkraftfunktion für die Distanz, die durch das Teilchen zurückgelegt wird.

6. Verfahren nach Anspruch 4 oder 5, wobei die Bewegungsvariable die Gesamtenergie $E(t)$ ist und der Kraftterm ist gleich

$$F(u(t), \eta(t)) = \frac{qBc^2}{E(t)} u(t) \times \widehat{B},$$

wobei $q$ die Ladung des Teilchens ist und $\hat{B}$ ein Einheitsvektor ist, derart, dass $B = B\hat{B}$.

7. Verfahren nach Anspruch 6, wobei das Finite-Differenzen-Verfahren den Einheitsrichtungsvektor wie folgt entwickelt:

$$u(t + \Delta t) = u(t) + \frac{2\alpha}{1 + \alpha^2}\left(u_\perp \times \widehat{B} - \alpha u_\perp\right),$$

wobei $u_\perp$ die Komponente des Einheitsrichtungsvektors senkrecht zu dem Magnetfeld ist und

$$\alpha = \frac{qBc^2 \Delta t}{E(t + \Delta t) + E(t)}.$$

8. Verfahren nach einem der vorstehenden Ansprüche, ferner umfassend:
Simulieren eines diskreten Wechselwirkungsereignisses, das zwischen zwei Zeitschritten des Finite-Differenzen-Verfahrens auftritt.

9. Verfahren nach Anspruch 8, wobei die simulierte Wechselwirkung eine Mehrfachstreuung einschließt.

10. Verfahren nach einem der vorstehenden Ansprüche, wobei das geladene Teilchen ein Elektron, ein Proton, ein Heliumion oder ein Kohlenstoffion ist.

11. Verfahren nach einem der vorstehenden Ansprüche, das als Teil einer Teilchentransportsimulation in einem zu bestrahlenden Medium gemäß einem strahlentherapeutischen Behandlungsplan ausgeführt wird.

12. Verfahren (100) zum Berechnen einer Dosisverteilung in einem zu bestrahlenden Medium gemäß einem Behandlungsplan, das Verfahren umfassend:

   Erhalten (110) eines Behandlungsplans, umfassend Anweisungen, die zum Steuern eines Strahlungsabgabesystems (300) geeignet sind;
   Durchführen (114) einer Teilchentransportsimulation, einschließlich:

   - Abtasten eines einfallenden Teilchens gemäß dem Behandlungsplan,
   - Simulieren von Wechselwirkungen zwischen dem Medium und dem Teilchen und
   - Simulieren weiterer Wechselwirkungen zwischen dem Medium und weiteren Teilchen, die durch diese Wechselwirkungen geschaffen werden,

   Berechnen (116) einer Dosisverteilung, die sich aus den simulierten Wechselwirkungen ergibt, wobei die Teilchentransportsimulation (114) das Ausführen des Verfahrens nach einem der vorstehenden Ansprüche einschließt.

13. Verfahren nach Anspruch 12, wobei die Teilchentransportsimulation eine Monte-Carlo-Typ-Simulation ist.

14. Verfahren nach Anspruch 12 oder 13, ferner umfassend:

   Berechnen (112) eines Fluenzfeldes auf der Basis des Behandlungsplans,
   wobei das einfallende Teilchen basierend auf dem Fluenzfeld abgetastet wird.

15. Behandlungsplanungssystem (200), umfassend einen Speicher (220) und eine Verarbeitungsschaltlogik (230), das konfiguriert ist, um das Verfahren nach einem der vorstehenden Ansprüche durchzuführen.

16. Computerprogramm (221), umfassend Anweisungen, die, wenn das Programm durch einen Computer ausgeführt wird, den Computer veranlassen, das Verfahren nach einem der Ansprüche 1 bis 14 vorzunehmen.

## Revendications

1. Procédé mis en œuvre par ordinateur permettant de faciliter un calcul de dose radiothérapeutique, le procédé étant configuré pour simuler un transport de particules chargées dans un champ magnétique externe $B$ selon un modèle où la seconde loi de Newton pour le vecteur de direction unitaire d'une particule chargée, $u'(t) = F(u(t), \eta(t))$, comprend un terme de force $F$ qui dépend du vecteur de direction unitaire $u(t)$ et dépend en outre de manière non linéaire d'une variable de mouvement $\eta(t)$ de la particule, le procédé comprenant :
   la résolution de la seconde loi de Newton à l'aide d'un procédé implicite de différences finies dans lequel, pour la durée d'un intervalle de temps, le terme de force est interpolé par sa valeur pour des valeurs interpolées du vecteur de direction unitaire et de la variable de mouvement, dans lequel la valeur interpolée de chaque quantité est une combinaison des valeurs de cette quantité aux points finaux $t,\ t + \Delta t$ de l'intervalle de temps.

2. Procédé selon la revendication 1, dans lequel au moins une des valeurs interpolées est une combinaison linéaire des valeurs de point final.

3. Procédé selon la revendication 2, dans lequel au moins une des valeurs interpolées est une moyenne des valeurs de point final.

4. Procédé selon l'une quelconque des revendications précédentes, dans lequel ladite au moins une variable de mouvement $\eta(t)$ de la particule est une ou plusieurs des variables parmi : une énergie totale, une énergie cinétique, une quantité de mouvement, une vitesse.

5. Procédé selon la revendication 4, dans lequel la valeur de la variable de mouvement au point final ultérieur, $\eta(t + \Delta t)$, est calculée en évaluant une fonction de puissance d'arrêt pour la distance parcourue par la particule.

6. Procédé selon la revendication 4 ou 5, dans lequel la variable de mouvement est une énergie totale $E(t)$, et le terme de force est égal à

$$F\big(\boldsymbol{u}(t), \eta(t)\big) = \frac{qBc^2}{E(t)} \boldsymbol{u}(t) \times \widehat{\boldsymbol{B}}$$,

où q est la charge de la particule et $\hat{\boldsymbol{B}}$ est un vecteur unitaire de telle sorte que $\boldsymbol{B} = B\hat{\boldsymbol{B}}$.

7. Procédé selon la revendication 6, dans lequel le procédé des différences finies fait évoluer le vecteur de direction unitaire comme suit :

$$\boldsymbol{u}(t + \Delta t) = \boldsymbol{u}(t) + \frac{2\alpha}{1 + \alpha^2}\big(\boldsymbol{u}_\perp \times \widehat{\boldsymbol{B}} - \alpha \boldsymbol{u}_\perp\big),$$

où $\boldsymbol{u}_\perp$ est la composante de vecteur de direction unitaire perpendiculaire au champ magnétique et

$$\alpha = \frac{qBc^2 \Delta t}{E(t + \Delta t) + E(t)}.$$

8. Procédé selon l'une quelconque des revendications précédentes, comprenant en outre :
la simulation d'un événement d'interaction discret se produisant entre deux intervalles de temps du procédé des différences finies.

9. Procédé selon la revendication 8, dans lequel l'interaction simulée comporte une diffusion multiple.

10. Procédé selon l'une quelconque des revendications précédentes, dans lequel la particule chargée est un électron, un proton, un ion d'hélium ou un ion de carbone.

11. Procédé selon l'une quelconque des revendications précédentes, qui est exécuté dans le cadre d'une simulation de transport de particules dans un milieu à irradier selon un plan de traitement radiothérapeutique.

12. Procédé (100) de calcul d'une distribution de dose dans un milieu à irradier selon un plan de traitement, le procédé comprenant :

l'obtention (110) d'un plan de traitement comprenant des instructions adaptées à la commande d'un système d'administration de rayonnement (300) ;
la réalisation (114) d'une simulation de transport de particules, y compris :

- l'échantillonnage d'une particule d'incident conformément au plan de traitement,
- la simulation des interactions entre le milieu et la particule, et
- la simulation d'autres interactions entre le milieu et d'autres particules créées par lesdites interactions,

le calcul (116) d'une distribution de dose résultant des interactions simulées, dans lequel ladite simulation de transport de particules (114) comporte l'exécution du procédé selon l'une quelconque des revendications précédentes.

13. Procédé selon la revendication 12, dans lequel la simulation de transport de particules est une simulation de type Monte-Carlo.

**14.** Procédé selon la revendication 12 ou 13, comprenant en outre :

le calcul (112) d'un champ de fluence sur la base du plan de traitement,
dans lequel la particule incidente est échantillonnée sur la base du champ de fluence.

**15.** Système de planification de traitement (200) comprenant une mémoire (220) et un ensemble de circuits de traitement (230) configuré pour réaliser le procédé selon l'une quelconque des revendications précédentes.

**16.** Programme informatique (221) comprenant des instructions qui, lorsque le programme est exécuté par un ordinateur, amènent l'ordinateur à mettre en œuvre le procédé selon l'une quelconque des revendications 1 à 14.

100

110

112

114

116

*Fig. 1*

200

210

220

221

230

*Fig. 2*

**Fig. 3**

**Fig. 4**

**x/z-plane**

*Fig. 5*

**y/z-plane**

*Fig. 6*

## Difference 1-P δ=0.3, 6 MeV, 1.5 T

*Fig. 7*

## Difference 2-P δ=0.3, 6 MeV, 1.5 T

*Fig. 8*

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Electron Transport in E and B Fields. **A. F. BIELAJEW et al.** Monte Carlo Transport of Electrons and Photons. Plenum Press, 1988
- **V. N. MALKOV et al.** Charged particle transport in magnetic fields in EGSnrc. *Med. Phys.*, 2016, vol. 43 (7), 4447-4458
- **F. SALVAT et al.** PENELOPE-2018: A Code System for Monte Carlo Simulations of Electron and Photon Transport. *OECD Nuclear Energy Agency, NEA/MB-DAV/R(2019)*, 2019, vol. 1
- Electron stopping powers for transport calculations. **M. J. BERGER et al.** Monte Carlo Transport of Electrons and Photons. Plenum Press, 1988